(19) Europäisches Patentamt
European Patent Office
Office européen des brevets

(11) **EP 3 319 625 B1**

(12) **FASCICULE DE BREVET EUROPEEN**

(45) Date de publication et mention
de la délivrance du brevet:
**01.01.2020 Bulletin 2020/01**

(21) Numéro de dépôt: **16734656.8**

(22) Date de dépôt: **04.07.2016**

(51) Int Cl.:
***A61K 39/00*** *(2006.01)*

(86) Numéro de dépôt international:
**PCT/EP2016/065733**

(87) Numéro de publication internationale:
**WO 2017/005702 (12.01.2017 Gazette 2017/02)**

(54) **PEPTIDES IMMUNOGENES DE PREPROCALCITONINE**

IMMUNOGENE PEPTIDE VON PRÄ-PROKALZITONIN

IMMUNOGENIC PREPROCALCITONIN PEPTIDES

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **09.07.2015 EP 15176174**

(43) Date de publication de la demande:
**16.05.2018 Bulletin 2018/20**

(73) Titulaires:
• **Institut Gustave Roussy**
**94805 Villejuif Cedex (FR)**
• **Institut National de la Santé Et de la Recherche Médicale**
**75654 Paris Cedex 13 (FR)**
• **Université Paris-Sud**
**91405 Orsay Cedex (FR)**

(72) Inventeurs:
• **MAMI-CHOUAIB, Fathia**
**92340 Bourg La Reine (FR)**
• **DURGEAU, Aurélie**
**91400 Orsay (FR)**

(74) Mandataire: **Plasseraud IP**
**66, rue de la Chaussée d'Antin**
**75440 Paris Cedex 09 (FR)**

(56) Documents cités:
**WO-A2-01/75179**

• **PAPEWALIS C ET AL: "Dendritic cell vaccination induces tumor epitope-specific Th1 immune response in medullary thyroid carcinoma", HORMONE AND METABOLIC RESEARCH, vol. 40, no. 2, février 2008 (2008-02), pages 108-116, XP008173300, ISSN: 0018-5043**

EP 3 319 625 B1

**Description**

La présente invention est relative à des combinaisons d'épitopes de la préprocalcitonine (ppCT) et à leur utilisation en immunothérapie antitumorale.

La vaccination ou immunothérapie peptidique est une approche thérapeutique qui fait actuellement l'objet d'un grand intérêt dans le cadre de la prévention ou du traitement des cancers. Son principe repose sur l'immunisation par des peptides reproduisant des épitopes T d'antigènes tumoraux reconnus par les lymphocytes T cytotoxiques (CTL), qui jouent un rôle majeur dans l'élimination des cellules cancéreuses exprimant ces antigènes à leur surface.

On rappellera que les CTL ne reconnaissent pas les antigènes protéiques entiers, mais des fragments peptidiques de ceux-ci, présentés par les molécules du complexe majeur d'histocompatibilité (CMH) exprimées à la surface de différentes cellules. Ce sont ces fragments peptidiques qui constituent les épitopes T.

La présentation de ces peptides résulte d'un processus complexe, dénommé « apprêtement de l'antigène », qui implique 3 étapes principales :

- la dégradation cytosolique des antigènes par un complexe multienzymatique dénommé protéasome ;
- la translocation des peptides issus de cette dégradation dans le réticulum endoplasmique (RE) par les transporteurs TAP ;
- l'association de ces peptides avec le CMH pour former des complexes stables peptide/CMH, qui seront exportés à la surface cellulaire.

Les épitopes présentés par le complexe majeur d'histocompatibilité de classe I (CMH I) ont généralement 8 à 11 acides aminés (aa) et sont reconnus par les cellules T CD8+, qui représentent la composante majeure de la réponse cytotoxique. Les épitopes présentés par le complexe majeur d'histocompatibilité de classe II (CMH II) ont généralement 13 à 18 acides aminés et sont reconnus par les cellules T CD4+.

L'identification de ces épitopes constitue une étape essentielle pour le développement de compositions d'immunothérapie anti-tumorale.

La préprocalcitonine est codée par le gène *CALCA* qui code également pour la forme alpha d'un neuropeptide, le « *calcitonin gene-related peptide* » ($\alpha$-CGRP). Ce gène contient 5 introns et 6 exons, et son transcrit primaire est l'objet d'un épissage alternatif tissu-spécifique. La jonction des exons 1, 2, 3 et 4 produit l'ARNm *calcitonine* dans les cellules C de la thyroïde, tandis que celle des exons 1, 2, 3, 5 et 6 produit l'ARNm $\alpha$-CGRP dans les neurones (MORRIS et al., Nature, 308, 746-8, 1984).

L'ARNm *calcitonine* code pour un précurseur de 141 acides aminés, la préprocalcitonine, qui contient une séquence signal N-terminale de 25 résidus, dont le clivage produit la procalcitonine, de 116 aa. La procalcitonine comprend une région N-terminale de 57 aa, suivie de la calcitonine mature, de 32 aa, et d'un peptide C-terminal de 21 aa, la katacalcine (ROSENFELD et al., Nature, 304, 129-35, 1983). L' $\alpha$-CGRP, sous sa forme mature, est un peptide de 37 aa, à effet vasodilatateur, retrouvé dans un grand nombre de tissus (ZAIDI et al., Crit Rev Clin Lab Sci, 28, 109-74, 1990). La séquence signal de la préprocalcitonine se retrouve également dans la préprohormone de l'$\alpha$-CGRP.

Le rôle physiologique de la calcitonine est principalement la protection du squelette pendant les périodes de « stress calcique », telles que la croissance, la grossesse et la lactation. La calcitonine est également produite en quantités importantes par les cellules des carcinomes médullaires de la thyroïde (CMT) et de certains carcinomes pulmonaires (COOMBES et al., Lancet, 1, 1080-3, 1974; MILHAUD et al., Lancet, 1, 462-3, 1974). Des niveaux élevés de calcitonine plasmatique sont des marqueurs diagnostiques et pronostiques dans ces tumeurs.

Il a été proposé d'utiliser des cellules dendritiques pulsées avec la calcitonine mature pour l'immunothérapie du CMT (SCHOTT et al., Cancer Immunol Immunother, 51, 663-8, 2002). Plus récemment, l'équipe des Inventeurs a identifié un peptide antigénique de 10 acides aminés issu du peptide signal de la préprocalcitonine. Ce peptide correspond à un épitope apprêté dans le réticulum endoplasmique, par un mécanisme indépendant du protéasome et des transporteurs TAP (Demande de brevet WO2009010874). La demande WO 01/75179 décrit l'identification, à l'aide d'un logiciel de prédiction de liaison à HLA, de peptides de la préprocalcitonine potentiellement capables de lier HLA.

Les Inventeurs ont maintenant identifié d'autres régions de la préprocalcitonine impliquées dans l'induction d'une réponse immune antitumorale, et montré que l'association de différents épitopes permet une meilleure réponse anti-tumorale.

La présente invention est telle que définie dans les revendications.

La présente invention a pour objet une composition comprenant une combinaison de séquences peptidiques choisie parmi les combinaisons suivantes :

- la combinaison des séquences SEQ ID Nos : 1, 2, 3, 5 et 6 ;
- la combinaison des séquences SEQ ID Nos : 1, 3, 4, 5 et 6 ; et
- la combinaison des séquences SEQ ID Nos : 1, 3, 5 et 6.

- MGFQKFSPFLALSIL (SEQ ID NO: 1), également dénommée ci-après ppCTI-15 ;
- EREGSSLDSPRSKRC (SEQ ID NO: 2), également dénommée ci-après ppCT71-85;
- GNLSTCMLGTYTQDF (SEQ ID NO: 3), également dénommée ci-après ppCT86-100 ;
- FLALSILVL (SEQ ID NO: 4), également dénommée ci-après ppCT9-17;
- VLLQAGSLHA (SEQ ID NO: 5), également dénommée ci-après ppCT16-25;
- LLAALVQDYV (SEQ ID NO: 6), également dénommée ci-après ppCT50-59.

Dans une composition selon l'invention, chacune desdites séquences est présente soit sous forme d'un peptide de 9 à 15 acides aminés, soit incluse dans un polypeptide multiépitopique chimérique. On définit ici un polypeptide chimérique comme une succession d'acides aminés qui n'est pas présente dans la nature. Un polypeptide chimérique tel qu'entendu ici comprend au moins deux séquences de ladite combinaison de séquences peptidiques telle que définie ci-dessus, lesdites séquences étant, dans ledit polypeptide, adjacentes, liées par un élément de liaison constitué par 1 à 5 acides aminés, ou séparées par une séquence comprenant un épitope d'une protéine autre que la préprocalcitonine.

Les compositions selon l'invention sont donc multiépitopiques, et sont de préférence capables de générer une réponse polyspécifique, au moins chez une partie des individus.

Les séquences SEQ ID Nos : 4 et 5 correspondent à un épitope apprêté dans le réticulum endoplasmique par un mécanisme indépendant du protéasome et des transporteurs TAP. Les séquences SEQ ID Nos : 3 et 6 correspondent à un épitope apprêté dans le réticulum endoplasmique par un mécanisme dépendant du protéasome et des transporteurs TAP. La composition selon l'invention comprend la séquence SEQ ID No : 6, soit sous forme de peptide constitué de cette séquence, soit incluse dans un polypeptide chimérique tel que défini ci-dessus.

Selon un mode de réalisation particulier de l'invention, la composition comprend un mélange de peptides de 9 à 15 acides aminés. Ce mélange peut être lyophilisé ou en suspension dans une solution adaptée à l'utilisation pharmaceutique.

Selon un autre mode de réalisation préféré de l'invention, la composition comprend un polypeptide chimérique comprenant ladite combinaison de séquences peptidiques telle que définie ci-dessus, lesdites séquences étant adjacentes, liées par un élément de liaison constitué par 1 à 5 acides aminés, ou séparées par une séquence comprenant un épitope d'une protéine autre que la préprocalcitonine. Le cas échéant, le polypeptide chimérique peut comprendre plusieurs copies d'une même séquence. Également, dans un polypeptide chimérique conforme à l'invention, au moins une partie des séquences immunogènes peut être insérée dans l'adénylate cyclase de *Bordetella pertussis* (CyA). Comme le récepteur de CyA est le même que celui de CD11b, et comme les cellules dendritiques expriment le récepteur CD11b, une telle construction permet de diriger directement un épitope immunogène vers les cellules dendritiques (DADAGLIO et al., Int. Immunol, 15, 1423-1430, 2003).

La composition selon l'invention comprend éventuellement au moins une autre séquence peptidique différente des séquences précédentes, notamment une séquence capable de se lier à une molécule HLA, par exemple HLA-B7, telle que l'une des séquences SPFLALSIL (SEQ ID NO : 19 ou ppCT7-15), EARLLLAAL (SEQ ID NO : 20 ou ppCT46-54), et/ou SPRSKRCGNL (SEQ ID NO : 21 ou ppCT79-88), la dite séquence peptidique étant sous forme d'un peptide isolé ou incluse dans un polypeptide multiépitopique chimérique selon l'invention.

Une composition multiépitopique conforme à l'invention peut comprendre, pour être largement utilisable sur une population dont les individus portent des allèles HLA différents, des épitopes présentés par différentes molécules du CMH.

Selon un mode de réalisation préféré de l'invention, la composition comprend en outre au moins un inhibiteur de point de contrôle immunitaire, tel que de façon non-limitative un anti-PD-1, un anti-PDL-1 ou un anti-CTLA4, notamment un anticorps, de préférence monoclonal, dirigé contre la molécule PD-1, PDL-1 ou CTLA4, de préférence la molécule humaine hPD-1, hPDL-1 ou hCTLA4. La composition selon l'invention comprend avantageusement un anti-PD-1, en particulier un anticorps monoclonal anti-PD-1, de préférence anti-hPD-1.

Un polypeptide chimérique conforme à la présente description peut être facilement obtenu par des méthodes connues en elles-mêmes, et notamment par les techniques classiques de l'ADN recombinant.

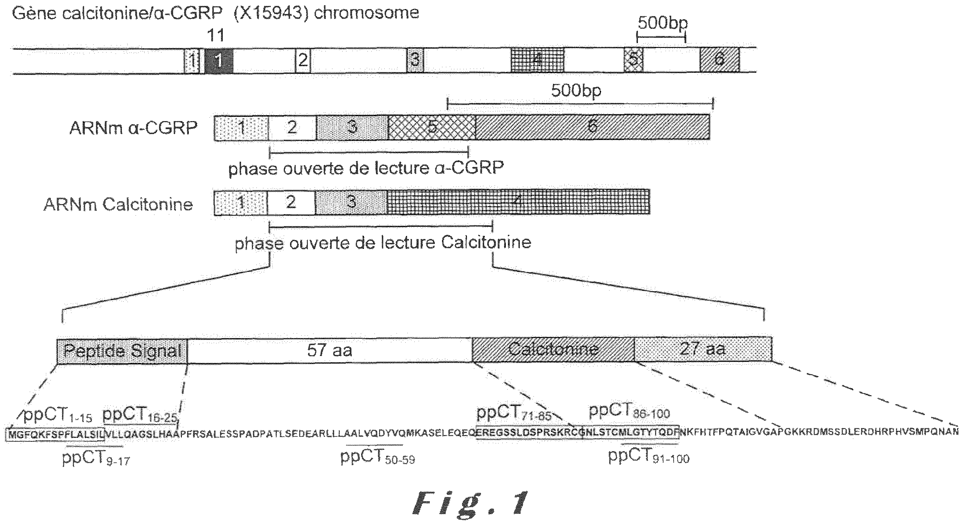

La position des peptides SEQ ID NO: 1-6 par rapport à la séquence complète de la préprocalcitonine est représentée sur la Figure 1.

A titre d'exemples non limitatifs de combinaisons de séquences présentes dans une composition selon l'invention, on citera les combinaisons comprenant les séquences précédentes et au moins un inhibiteur de point de contrôle immunitaire tel que défini ci-dessus, en particulier un anticorps monoclonal anti-PD-1, de préférence un anti-hPD-1, et/ou l'une des séquences SEQ ID NO : 19, 20, et/ou 21.

La présente invention a également pour objet une composition comprenant au moins une molécule d'acide nucléique codant pour un mélange de peptides immunogènes ou pour un polypeptide chimérique tels que définis ci-dessus. Ces polynucléotides peuvent être insérés dans un vecteur d'expression, sous contrôle transcriptionnel d'un promoteur approprié, pour permettre l'expression du peptide immunogène ou du polypeptide chimérique conforme à l'invention dans une cellule ou un organisme hôte. Le choix du vecteur d'expression dépend notamment de la cellule ou de l'organisme (procaryote ou eucaryote) où l'expression est souhaitée. S'il est prévu d'administrer directement le polynucléotide à un patient à traiter, on utilisera de préférence un plasmide d'ADN nu, ou un vecteur permettant une expression transitoire, par exemple un vecteur dérivé d'un adénovirus ou d'un virus de la vaccine..

D'autres compositions conformes à l'invention peuvent également comprendre des cellules dendritiques, chargées avec un peptide ou une composition multiépitopique de l'invention, ou transformées avec un polynucléotide de l'invention, inséré dans un vecteur d'expression approprié. Elles peuvent également comprendre des cellules présentatrices de l'antigène artificielles chargées avec un peptide ou une composition multiépitopique de l'invention. Ces cellules présentatrices de l'antigène artificielles peuvent être notamment des vésicules dérivées de cellules tumorales (texosomes) décrites dans la Demande PCT WO 1999/003499, ou des exosomes dérivés de cellules dendritiques, comme décrit dans la publication de VIAUD et al. (J Immunother, 34, 65-75, 2011).

Un autre aspect de la présente invention est l'utilisation des compositions décrites plus haut comme médicament, en particulier comme médicament destiné à l'immunothérapie anti-tumorale, plus particulièrement au traitement de tumeurs exprimant la calcitonine et/ou l'$\alpha$-CGRP. Ceci englobe en particulier les carcinomes pulmonaires, à petites cellules ou non, ainsi que les carcinomes médullaires de la thyroïde. Les compositions selon l'invention peuvent également être utilisées pour le traitement de pathologies associées à un taux sérique élevé de calcitonine ou procalcitonine ou pro $\alpha$-CGRP, telles que les pathologies cancéreuses suivantes : cancer du rein, du sein, gastrointestinal (TABOLLI et al, Tumori, 69, 227-230, 1983), du pancréas, de la prostate (SIM et al, Ann Clin Lab Sci., 26, 487-95, 1996), du foie (CONTE et al, Acta Endocrinol, 106, 109-11, 1984) ou des leucémies myélocytaires chroniques (TAKUBO et al Haematologia, 31, 177-9, 2001), des leucémies aigües indifférenciées et myéloblastiques (KIEFER et al, Leuk Lymphoma., 13, 501-507, 1994), tumeurs neuroendocrines et cancer du foie (GHILLIANI et al, Cancer Res, 49, 6845-6851, 1989).

Une composition selon l'invention peut être utilisée de façon particulièrement avantageuse pour l'immunothérapie d'une tumeur dont les cellules n'expriment pas les transporteurs de peptides TAP.

Les compositions décrites plus haut sont particulièrement appropriées au traitement d'un patient HLA-A*0201.

Lesdits médicaments comprennent, en tant que principe actif, une composition conforme à l'invention. Lesdits médicaments peuvent comprendre en outre les excipients usuels, ainsi que des adjuvants habituellement utilisés en immunothérapie, et permettant par exemple de favoriser l'administration du principe actif, de le stabiliser, d'augmenter son immunogénicité, etc. Des exemples d'adjuvants utilisables incluent les oligodéoxynucléotides CpG, l'Apoptosis-Inducing Factor (AIF), les Heat Shock Proteins (HSP), les récepteurs Toll-like (TLRs) tel que des agonistes de TLR3 (Poly I:C), et des cytokines et chimiokines telles que l'IL-7, l'IL-12, l'IL-15 et le GM-CSF et CCL5 (Rantes). Selon un mode de réalisation préféré de la présente invention, lesdits médicaments sont des vaccins, en particulier des vaccins thérapeutiques.

La présente invention sera mieux comprise à l'aide du complément de description qui va suivre, qui se réfère à des exemples illustrant l'identification de mélanges de peptides immunogènes conformes à l'invention.

## LISTE DES FIGURES

Figure 1 : Position des peptides SEQ ID NO: 1-7 par rapport à la séquence complète de la préprocalcitonine.

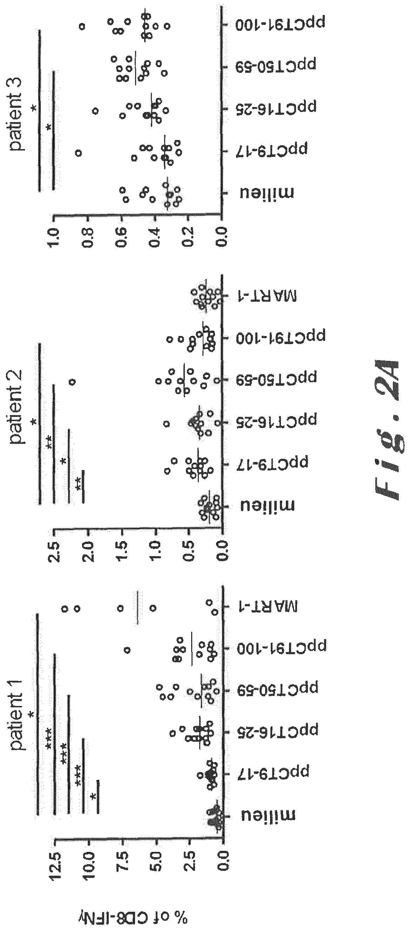

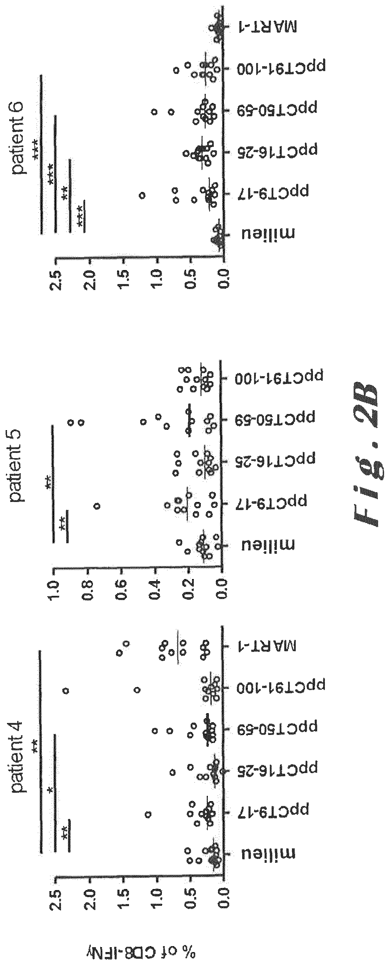

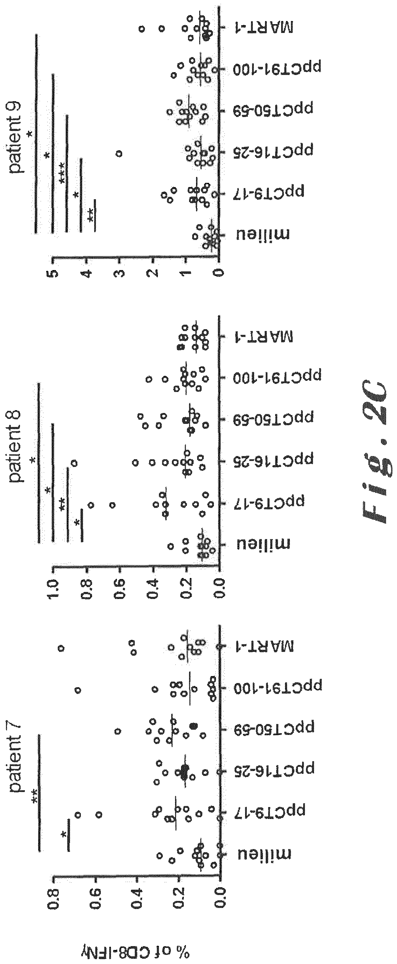

Figure 2 : Stimulation *in vitro* de PBMC de patients avec les peptides de SEQ ID NO : 4-7. A à C : Les PBMC des 9 patients atteints de cancer bronchique non à petites cellules (CBNPC) ont été stimulés avec chacun des 4 peptides puis le nombre de cellules CD8$^+$/IFN$\gamma^+$ a été analysé par cytométrie de flux. Chaque point (n) correspond à 8 puits indépendants de stimulation. La médiane de ces points est représentée pour chaque patient et chaque peptide (n=12). Les augmentations statistiquement significatives sont indiquées (*p<0.05 ; **p<0.01 ; ***p<0.005).

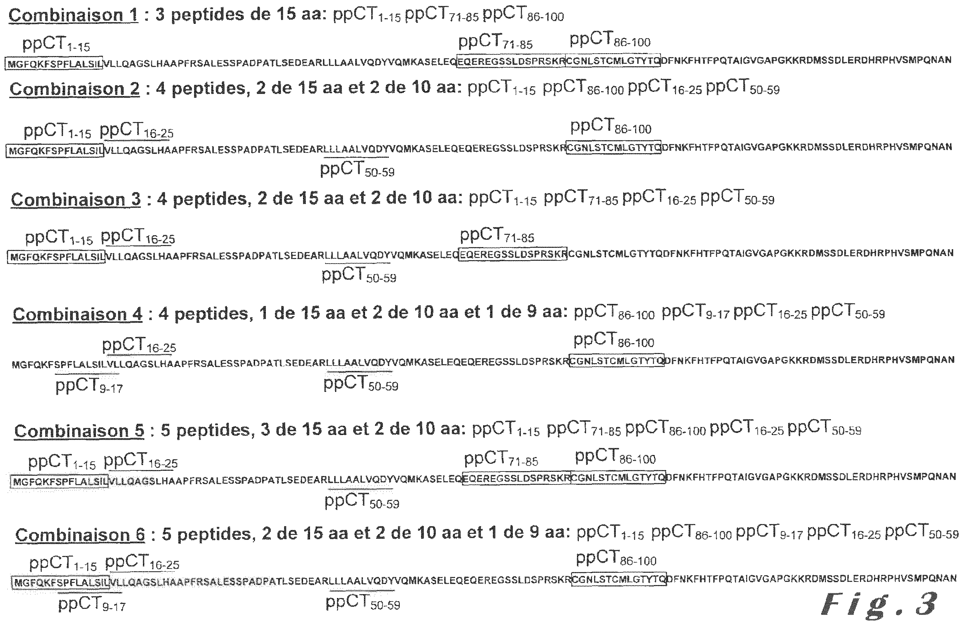

Figure 3 : Résumé des différentes combinaisons peptidiques testées *in vitro* et *in vivo* dans un modèle de souris transgéniques pour les molécules HLA-A2.

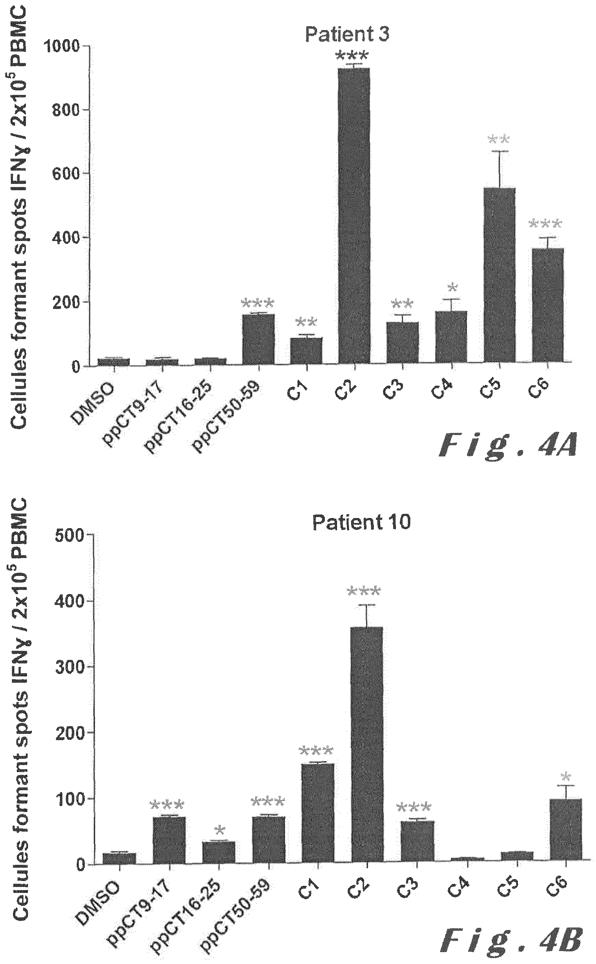

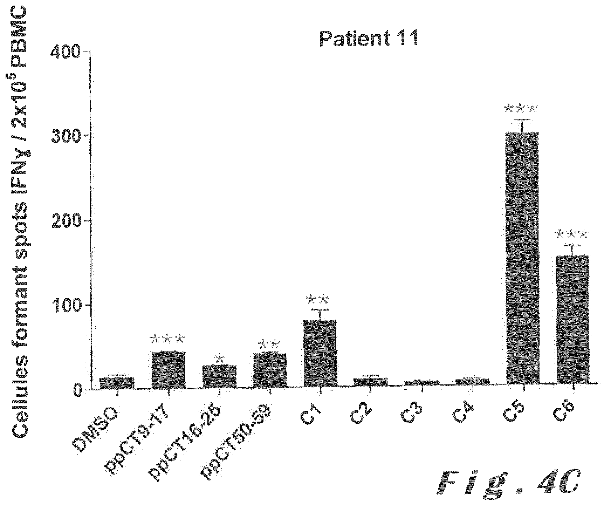

Figure 4 : Stimulation *in vitro* des PBMC de patients avec les peptides courts de 9-10 aa ou les différentes combinaisons peptidiques. A à C : Les PBMC des patients 3 (A), 10 (B) et 11 (C) ont été stimulés soit avec un des peptides SEQ ID Nos : 4 à 6, seul, soit avec une des combinaisons 1 à 6 décrites en figure 3 ; le nombre de cellules sécrétant de l'IFN$\gamma^+$ a ensuite été analysé par ELISPOT. Les augmentations statistiquement significatives sont indiquées (*p<0.05 ; **p<0.01 ; ***p<0.005).

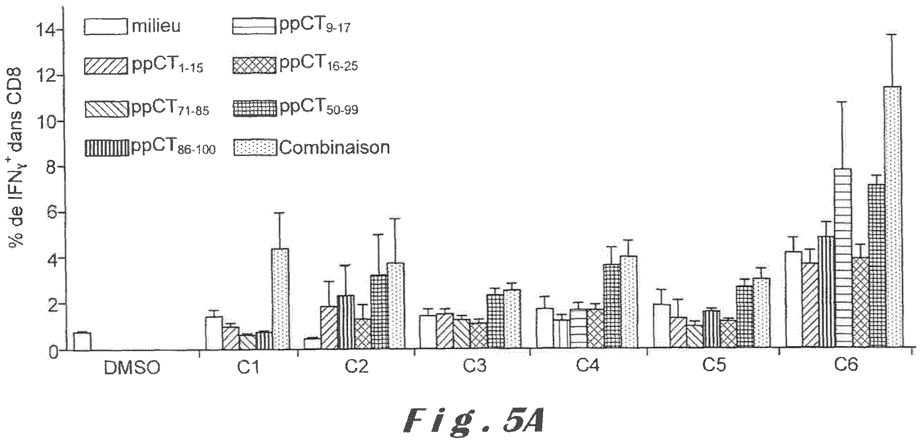

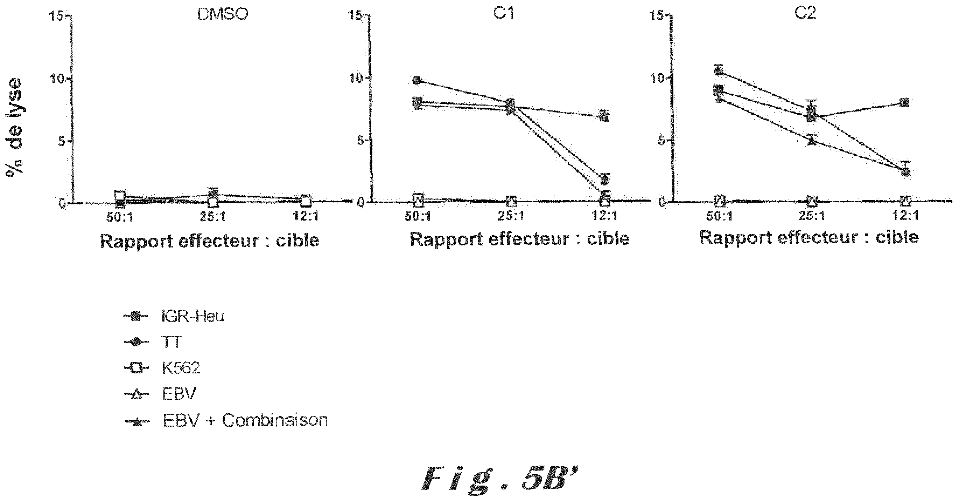

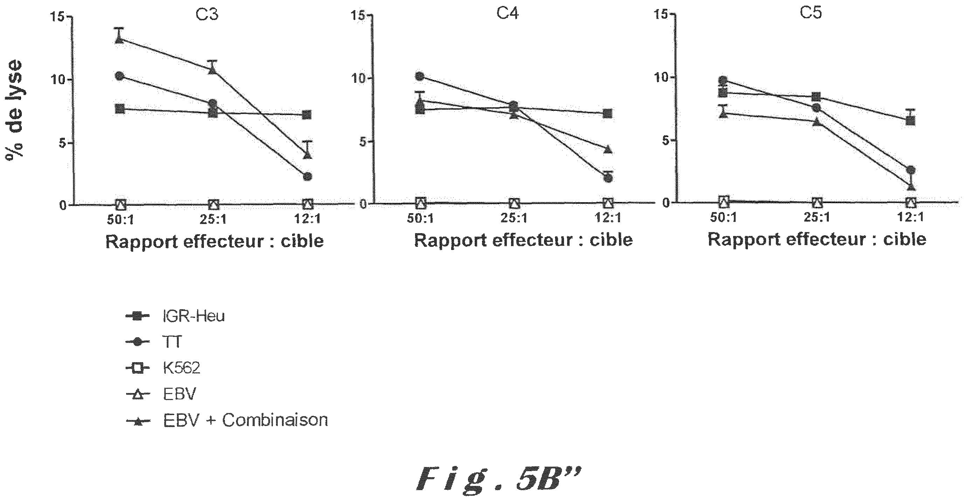

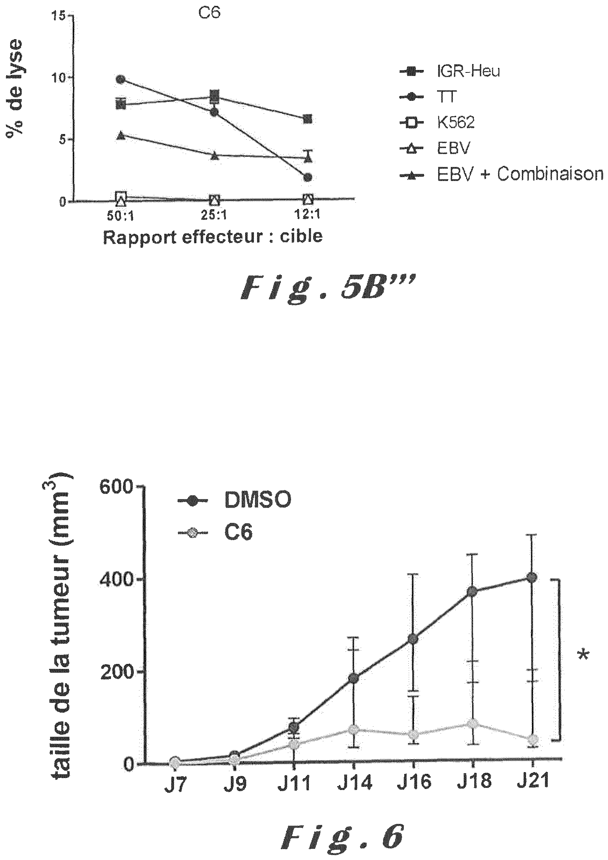

Figure 5 : Immunisation des souris transgéniques pour les molécules HLA-A2 avec les différentes combinaisons peptidiques **A.** Pourcentage de lymphocytes T CD8$^+$/IFN$\gamma^+$ après restimulation *ex vivo* des splénocytes avec chacun des peptides seul ou en combinaison. **B', B"** et B'''. Réponse cytotoxique des lymphocytes T CD8 après immunisation des souris avec les différentes combinaisons peptidiques (C1-C6).

Figure 6 : Effet antitumoral de la combinaison peptidique C6 dans le modèle de souris transgénique pour les molécules HLA-A2. 1x10⁶ cellules tumorales D122-ppCT ont été greffées en sous-cutané dans le flanc des souris transgénique à J0. Les souris ont ensuite été vaccinées en sous-cutané à J0, J3, J4 et J14, avec 100$\mu$M de chaque peptide composant la combinaison C6 et de 25 $\mu$g d'adjuvant. La taille de la tumeur est définie par la formule longueur*largeur*profondeur. La différence de croissance tumorale statistiquement significative est indiquée (*p<0.05).

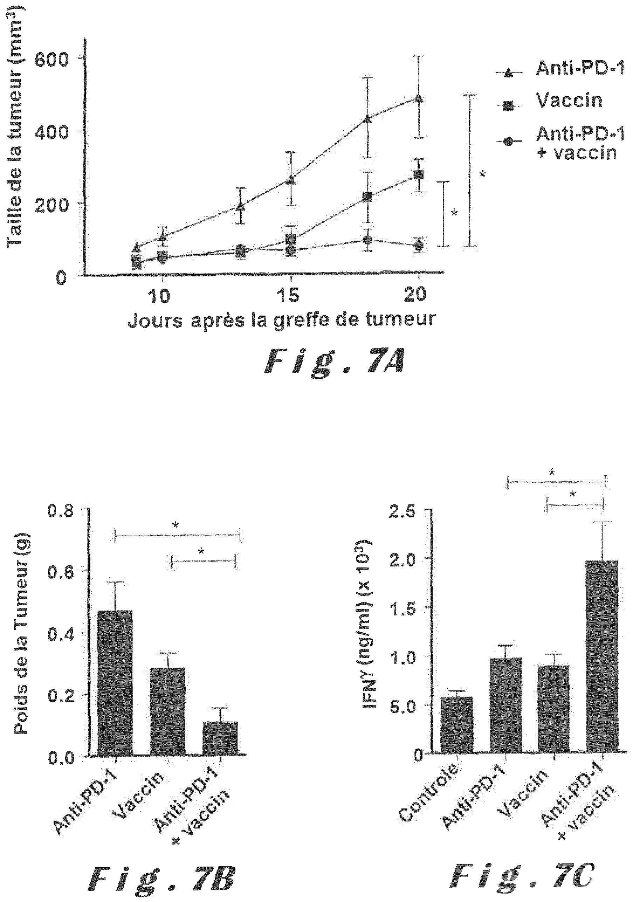

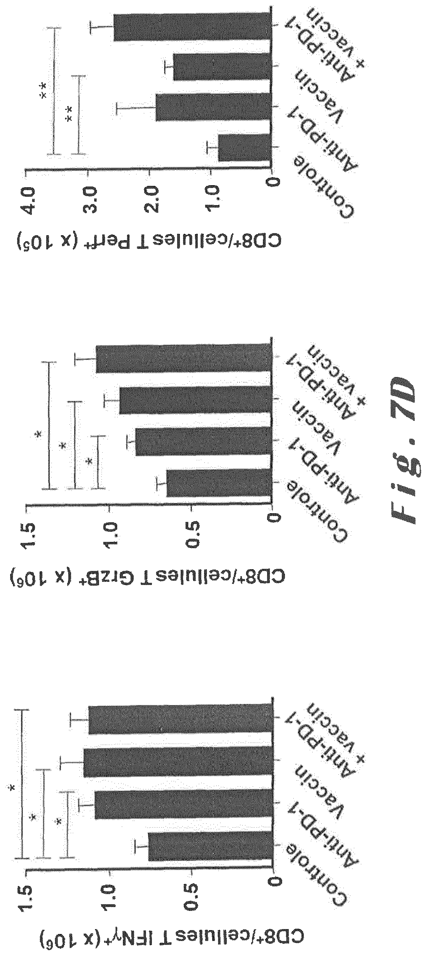

Figure 7 : Potentialisation de la réponse anti-tumorale et du contrôle de la croissance de la tumeur par l'immunothérapie combinée. **A.** La thérapie combinée optimise le contrôle de la croissance tumorale. Des souris NOD-*scid Il2r$\gamma$^null* (NSG) ont reçu une greffe de fragments de tumeur Heu-nIR dans le flanc, puis un transfert adoptif de PBMC humains de donneurs sains à J10, suivi à J11 par une vaccination intraveineuse avec le vaccin à base du mélange de peptides C6, combiné ou non avec un anticorps monoclonal anti-PD1 à partir de J12. La croissance tumorale a été enregistrée tous les deux jours jusqu'à la fin de l'expérience. **B.** Les souris traitées avec la thérapie combinée (vacccin + anti-PD-1) ont montré la plus forte réduction du poids de la tumeur, comparé aux souris traitées par le vaccin seul (*p < 0,04) ou l'anti-PD-1 seul (*p < 0,03). C. Production de cytokine induite par la thérapie combinée *in vivo*. Les taux plasmatiques d'IFN-$\gamma$ humain dans le sérum des souris traitées ou non-traitées ont été déterminés par ELISA avant la fin de l'expérience (*p < 0,01). **D.** Production d'IFN-$\gamma$, de granzyme B (GrmB) et de perforine (Perf) par les splénocytes des souris traitées et non traitées. Les cellules T CD8+ produisant de L'IFN-$\gamma$, du granzyme B (GrmB) et de la perforine (Perf) ont été évaluées par analyses de la fluorescence intracellulaire. Les valeurs correspondent à l'intensité moyenne de fluorescence (MFI). *p < 0,05 ; **p < 0,001. Les barres d'erreurs représentent l'erreur type de la moyenne (SEM).

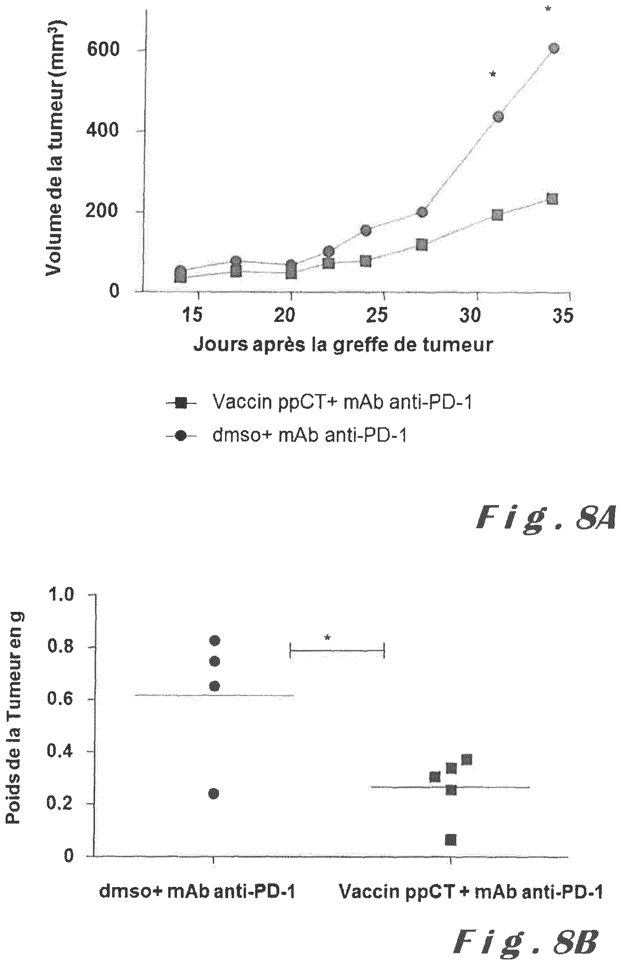

Figure 8 : Potentialisation du contrôle de la croissance de la tumeur par l'immunothérapie combinée. **A.** La thérapie combinée optimise le contrôle de la croissance tumorale. Des souris NOD-*scid Il2r$\gamma$^null* (NSG) ont reçu une greffe de fragments de tumeur Heu-nIR dans le flanc, puis un transfert adoptif de PBMC humains de donneurs sains à J10, suivi à J11 par une vaccination intraveineuse avec le vaccin à base du mélange de peptides C6a, combiné ou non avec un anticorps monoclonal anti-PD1 à partir de J12. La croissance tumorale a été enregistrée tous les deux jours jusqu'à la fin de l'expérience. **B.** Les souris traitées avec la thérapie combinée (vacccin + anti-PD-1) ont montré la plus forte réduction du poids de la tumeur, comparé aux souris traitées par l'anti-PD-1 seul.

**EXEMPLE 1: STIMULATION *IN VITRO* DES PBMC DE PATIENTS PAR PEPTIDES DE 9 OU 10 ACIDES AMINES DE LA PREPROCALCITONINE.**

**MATERIEL ET METHODES**

**Peptides**

Les peptides suivants ont été choisis:

ppCT9-17 FLALSILVL (SEQ ID NO:4) ;
ppCT16-25 VLLQAGSLHA (SEQ ID NO:5).
ppCT50-59 LLAALVQDYV (SEQ ID NO:6) ;
ppCT91-100 CMLGTYTQDF (SEQ ID NO:7) ;

**Cellules utilisées et stimulation**

Les PBMC (*peripheral blood mononuclear cells*) de 11 patients atteints de cancer bronchique non à petites cellules (CBNPC) ont été isolés par Ficoll à partir du sang périphérique. Pour chaque peptide, des PBMC ont été stimulés 2 fois (J0 et J7) avec 20 $\mu$M de peptide. Les PBMC ont été cultivés pendant 2 semaines en plaques 96 puits, à raison de 20 millions de cellules / plaque, en milieu complet (RPMI1640, 10% SAB, 1% sodium pyruvate, 0.1% pénicilline/streptavidine) en présence d'IL-2 (20 UI/ml) + IL-4 (10 ng/ml) + IL-7 (10 ng/ml).

**Analyse par cytométrie**

Après 15 jours, les PBMC sont de nouveau stimulés avec 2,5 $\mu$M du peptide utilisé lors de la première étape de stimulation, en présence de 100 $\mu$g/ml de Brefeldin A, et incubés pendant 6h à 37°C, 5% $CO_2$. Les cellules sont

ensuite incubées 20 min. avec un anticorps anti-CD8-APC, puis lavées en PBS, fixées avec PBS-formaldéhyde 2% et perméabilisées par BSA/saponine. Elles sont ensuite incubées avec un anticorps anti-IFNγ-PE. L'analyse est réalisée par cytométrie en flux. L'étude statistique est réalisée par le test de Mann-Whitney et les augmentations statistiquement significatives sont indiquées (*p<0.05 ; **p<0.01 ; ***p<0.005).

## RESULTATS

Les résultats sont illustrés dans la Figure 2.

L'analyse par cytométrie en flux de la réponse à la stimulation avec les différents peptides de 9 ou 10 acides aminés a permis de montrer que la stimulation par les peptides ppCT9-17, ppCT16-25, ppCT50-59 et ppCT91-100 permet une augmentation de 1 à 4,5 fois le niveau basal des lymphocytes T CD8$^+$/INFγ$^+$ chez les patients testés. L'analyse de différents puits de stimulation a permis de montrer que cette augmentation de la réponse CD8 est statistiquement significative (*p<0.05 ; **p<0.01 ; ***p<0.005). Néanmoins, les différents patients ne répondent pas de la même manière à chacun des peptides. Les patients 1 ; 2 ; 6 ; 8 et 9 déclenchent une sécrétion d'IFNy par les lymphocytes T CD8 après stimulation par l'ensemble des peptides testés, alors que les patients 4, 5 et 7 ne déclenchent une réponse lymphocytaire qu'après stimulation avec les peptides ppCT9-17 et ppCT50-59 tandis que le patient 3 déclenche une réponse avec les peptides ppCT50-59 et ppCT91-100.

En conclusion, il apparait que les peptides sont immunogènes selon les patients testés et permettent une réponse immunitaire *via* les lymphocytes T CD8. Vu la variabilité de la réponse aux différents peptides, une combinaison peptidique aurait plus de chance d'induire une réponse anti-ppCT chez la majorité des patients inclus.

## EXEMPLE 2 : STIMULATION *IN VITRO* DE PATIENTS AVEC LES PEPTIDES COURTS DE 9-10 AA OU LES DIFFERENTES COMBINAISONS PEPTIDIQUES

## MATERIEL ET METHODES

### Peptides

Les peptides suivants ont été choisis:

ppCT1-15 : MGFQKFSPFLALSIL (SEQ ID NO: 1) ;
ppCT71-85: EREGSSLDSPRSKRC (SEQ ID NO: 2) ;
ppCT86-100: GNLSTCMLGTYTQDF (SEQ ID NO: 3) ;
ppCT9-17 FLALSILVL (SEQ ID NO:4) ;
ppCT16-25 VLLQAGSLHA (SEQ ID NO:5).
ppCT50-59 LLAALVQDYV (SEQ ID NO:6) ;
ppCT91-100 CMLGTYTQDF (SEQ ID NO:7) ;

### Cellules utilisées et stimulation

Les PBMC (*peripheral blood mononuclear cells*) de 3 patients atteint de cancer du poumon non à petites cellules ont été isolés par Ficoll à partir du sang périphérique, puis stimulés 2 fois (J0 et J7) avec 20 μM de chacun des peptides ou avec les combinaisons équimolaire de 20 μM de chaque peptide décrites dans la figure 3. Les PBMC sont cultivés pendant 2 semaines en plaques 96 puits, à raison de 20 millions de cellules / plaque, en milieu complet (RPMI1640, 10% SAB, 1% sodium pyruvate, 0.1% pénicilline/streptavidine) en présence d'IL-2 (20 UI/ml) + IL-4 (10 ng/ml) + IL-7 (10 ng/ml).

### Analyse par ELISPOT

Après 15 jours, les PBMC sont récupérés puis sont incubés sur des plaques ELISPOT à raison de 100 000 cellules par puits, en présence de milieu avec 2,5 μM de chaque peptide ou avec les combinaisons équimolaires de 2,5 μM de chaque peptide utilisé pour la stimulation. Les plaques sont incubées 15 à 18h à 37°C et 5% de $CO_2$. Les spots IFNγ sont révélés suivant les instructions du fabriquant (Gen-Probe Diaclone). Les spots formés par les cellules sécrétant de l'IFNγ sont comptés et analysés avec le C.T.L Immunospot system (Cellular Technology Ltd). L'étude statistique est réalisé par le test de Mann-Whitney et les augmentations statistiquement significatives sont indiquées (*p<0.05 ; **p<0.01 ; ***p<0.005)

**RESULTATS**

Les résultats sont illustrés dans la Figure 4.

L'analyse du nombre de cellules sécrétrices d'IFNy suite à la stimulation des PBMC de 3 patients avec les combinaisons décrites en figure 3 a permis de montrer que ces différentes combinaisons permettent de stimuler et ainsi d'amplifier les lymphocytes spécifiques de ces peptides. En effet, une augmentation significative du nombre de cellules sécrétrices d'IFNy est observée pour 3 combinaisons chez le patient 11, 4 combinaisons chez le patient 10 et les 6 combinaisons chez le patient 3.

De plus, les stimulations par les combinaisons C2, C5 et C6 augmentent également le nombre de cellules sécrétant de l'IFNγ par rapport aux stimulations par les peptides seuls. Ces réponses sont patient-dépendantes. En effet, la stimulation des PBMC du patient 3 par les combinaisons C2, C5 et C6 permettent d'augmenter le nombre de cellules sécrétant de l'IFNγ de 6 fois pour C2, 3 fois pour C5 et 2 fois pour C6, alors que pour le patient 10, seule la stimulation des PBMC avec la combinaison C2 augmente le nombre de cellules sécrétant de l'IFNγ de 3 fois et la stimulation des PBMC du patient 11 par les combinaisons C5 et C6 permettent d'augmenter le nombre de cellules sécrétant de l'IFNγ de 3 fois pour C5 et 2 fois pour C6.

Ce résultat démontre que la combinaison de plusieurs peptides issus de l'antigène ppCT permet une augmentation de la réponse immunitaire développée par les lymphocytes T CD8. De plus, ces résultats démontrent également l'intérêt de l'utilisation d'une combinaison peptidique par rapport aux peptides seuls pour déclencher une réponse immunitaire chez la majorité des patients.

**EXEMPLE 3: IMMUNISATION DE SOURIS TRANSGENIQUES AVEC LES DIFFERENTES COMBINAISONS PEPTIDIQUES**

**MATERIEL ET METHODES**

**Peptides**

Les peptides suivants ont été choisis:

ppCT1-15 : MGFQKFSPFLALSIL (SEQ ID NO: 1) ;
ppCT71-85: EREGSSLDSPRSKRC (SEQ ID NO: 2) ;
ppCT86-100: GNLSTCMLGTYTQDF (SEQ ID NO: 3) ;
ppCT9-17 FLALSILVL (SEQ ID NO:4) ;
ppCT16-25 VLLQAGSLHA (SEQ ID NO:5).
ppCT50-59 LLAALVQDYV (SEQ ID NO:6) ;
ppCT91-100 CMLGTYTQDF (SEQ ID NO:7) ;

**Cellules utilisées**

Les lignées cellulaires tumorales humaines TT (issue d'une tumeur médullaire de la thyroïde) et IGR-Heu (isolée à partir de la tumeur CBNPC du patient Heu), exprimant l'antigène ppCT, sont utilisées comme cellules cibles. La lignée K562 (issue d'un patient atteint d'une leucémie chronique myéloïde) est utilisée comme contrôle négatif. La lignée Heu-EBV, (issue des lymphocytes B du patient Heu) est utilisée pour déterminer la spécificité de la lyse. Après marquage avec le $^{51}$Cr, les cellules Heu-EBV sont incubées avec le mélange équimolaire à 20 $\mu$M de chaque peptide (décrit dans la Figure 4).

**Immunisation des souris**

Les souris transgéniques pour les molécules HLA-A2 ont été immunisées 4 fois (J0, J7, J14 et J21) en sous-cutané au niveau du flanc avec 100 $\mu$l de combinaisons peptidiques contenant un mélange équimolaire de 100 $\mu$M de chacun des peptides et d'un adjuvant (25 $\mu$g de Poly (I:C)) (Figure 4). 28 jours après la première injection, les souris ont été sacrifiées puis les splénocytes ont été récupérés et cultivés une nuit en milieu complet (RPMI1640, 10% SVF, 1% sodium pyruvate, 0,1% pénicilline/streptavidine) en présence d'IL-2 (20 UI/ml).

**Analyse par cytométrie et analyses par test de cytotoxicité**

Le lendemain, les splénocytes ont été stimulés avec 2,5 $\mu$M de chacun des peptides, seul ou en combinaison, en présence de 10 $\mu$g/ml de Brefeldin A et incubés pendant 6 h à 37°C, 5% $CO_2$. Les cellules sont ensuite incubées 20 min avec un anticorps anti-CD8-PE, puis lavées en PBS, fixées avec PBS-formaldéhyde 2% et perméabilisées avec BSA/saponine. Elles sont ensuite incubées avec un anticorps anti-IFNγ-APC. L'analyse est réalisée par cytométrie en flux.

Pour l'analyse de l'activité cytotoxique, les lymphocytes T CD8 ont été isolés des splénocytes par déplétion négative (miltenyi biotech). L'activité cytotoxique des lymphocytes T CD8 ainsi obtenus a été évaluée par un test de relargage de chrome 51 ($^{51}$Cr). Les cellules cibles (décrites dans le paragraphe ci-dessus) sont incubées 1h à 37°C en présence de 20 $\mu$M de $^{51}$Cr. Les cellules sont ensuite lavées avec du RPMI avant d'être co-cultivées à 37°C avec les lymphocytes T CD8 à des rapports effecteur/cible de 50:1, 25:1 et 12:1. Après 4h d'incubation, la concentration de $^{51}$Cr relargué dans le surnageant des co-cultures est mesurée. Le pourcentage de lyse est calculé grâce à la formule suivante :

$$\% \ de \ lyse = \frac{(\ Chrome \ relagué - Chrome \ relagué \ minimun)}{(Chrome \ relagué \ maximun - Chrome \ relagué \ minimun)} X \ 100$$

## RESULTATS

L'analyse par cytométrie en flux des splénocytes isolés suite à l'immunisation des souris avec les combinaisons peptidiques C1 à C6 montre une augmentation du pourcentage de lymphocytes T CD8 exprimant de l'IFNγ (Figure 5A). Cette augmentation est dépendante de la combinaison utilisée, allant d'un fold change de 3 à 13 (4,8 pour C1 ; 3 pour C2, 3,4 pour C3, 5,5 pour C4, 4,3 pour C5 et 13,6 pour C6). Ces résultats montrent que l'immunisation des souris transgéniques avec les différentes combinaisons peptidiques permet de développer des réponses immunitaires via l'induction de lymphocytes T CD8.

De plus, la stimulation *ex vivo* avec chacun des peptides issus de la combinaison permet de démontrer l'effet additionnel des combinaisons. Pour la combinaison C1, on observe que la réponse après stimulation *ex vivo* augmente d'un fold change de 4,2 ; 5,2 et 4,6 par rapport aux stimulations *ex vivo* par les peptides ppCT1-15 ; ppCT71-85 et ppCT86-100 respectivement. La même observation peut être faite sur l'efficacité de la réponse immunitaire via les lymphocytes T CD8 pour l'ensemble des combinaisons. Néanmoins, aucune des combinaisons ne montre d'effet sur le peptide ppCT50-59, dû notamment à la forte réponse qu'il induit naturellement.

Enfin, les lymphocytes T CD8 générés par les immunisations des souris avec les combinaisons peptidiques sont capables de reconnaitre et de lyser de façon spécifique les cellules exprimant l'antigène ppCT, comme IGR-Heu et TT (Figure 5B). En effet, la figure 5B montre que les lymphocytes T CD8 issus de splénocytes de souris non immunisées ne sont pas capables de lyser ni la lignée tumorale IGR-Heu ni la lignée tumorale TT, contrairement aux lymphocytes T CD8 issus de splénocytes de souris immunisées. Suivant les conditions d'immunisation, les lymphocytes lysent entre 7 et 15% des cellules exprimant l'antigène ppCT. Les lymphocytes T ne lysent pas les cellules K562 ou les cellules Heu-EBV sauf si celles-ci sont chargées avec la combinaison peptidique. Ce résultat montre la spécificité de la lyse obtenue pour les cellules tumorales exprimant l'antigène ppCT.

L'ensemble des résultats, obtenus par l'immunisation des souris transgéniques, démontre que l'utilisation de combinaisons peptidiques permet d'activer et d'augmenter la réponse immunitaire exercée par les lymphocytes T CD8 contre les cellules exprimant l'antigène ppCT.

## EXEMPLE 4: VACCINATION DES SOURIS TRANSGENIQUES AVEC LA COMBINAISON C6

### MATERIEL ET METHODES

### Peptides

Les peptides suivants ont été choisis:

    ppCT1-15 : MGFQKFSPFLALSIL (SEQ ID NO: 1) ;
    ppCT86-100: GNLSTCMLGTYTQDF (SEQ ID NO: 3) ;
    ppCT9-17 FLALSILVL (SEQ ID NO:4) ;
    ppCT16-25 VLLQAGSLHA (SEQ ID NO:5) ;
    ppCT50-59 LLAALVQDYV (SEQ ID NO:6).

### Cellules utilisées

La lignée murine LL2 (Lewis lung carcinoma) transgénique pour les molécules HLA-A2 (lignée D122) a été infectée avec un lentivirus exprimant la GFP et qui code pour l'antigène humain ppCT. $10^6$ cellules ont ensuite été injectées en sous cutané au niveau du flanc des souris.

### Immunisation des souris

Les souris transgéniques pour les molécules HLA-A2 ont été vaccinées 4 fois (J0, J4, J7 et J14) en sous-cutané au niveau du flanc avec 100 $\mu$l de la combinaison peptidique C6 contenant un mélange équimolaire de 100 $\mu$M de chacun des peptides et d'un adjuvant (25 $\mu$g de Poly (I:C)).

### Suivi de la croissance tumorale

Après la greffe des cellules tumorales, le poids des souris a été suivi tous les jours, et les tumeurs ont été mesurées tous les 2-3 jours à partir du 7$^{eme}$ jour. La taille est calculée en mm$^3$ de la façon suivante : Longueur x largeur x profondeur. La différence de croissance tumorale statistiquement significative est calculée par un t test et indiquée (*p<0.05).

### RESULTATS

Dans le modèle de souris transgénique pour les molécules HLA-A2 ayant une tumeur exprimant la ppCT, la vaccination avec la combinaison C6 induit une réduction de la croissance tumorale de façon significative par rapport aux souris non vaccinées. En effet, au bout de 21 jours, seule une souris vaccinée sur 3 possède une tumeur d'une taille de 190 mm$^3$, alors que les 3 souris contrôles ont développé une tumeur avec une taille moyenne de 350mm$^3$ (Figure 6).

Ce résultat montre la capacité de la vaccination avec la combinaison C6 à générer une réponse immunitaire efficace contre les tumeurs exprimant la ppCT, démontrant ainsi l'intérêt de l'utilisation de ces combinaisons en immunothérapie anti-tumorale.

### EXEMPLE 5 : VACCINATION DES SOURIS HUMANISÉES AVEC LA COMBINAISON C6 SEULE OU COMBINÉE A UN ANTI-PD-1

### MATERIEL ET METHODES

### Souris, greffe de tumeur humaine et transfert de PBMC humains

Des souris *NOD-scid Il2r$\gamma^{null}$* (NSG ; Laboratoire Jackson) âgées de 3-4 semaines ont été greffées en sous-cutané avec la tumeur humaine Heu-nIR, générée par implantation de la lignée de cellules tumorales humaines IGR-Heu préalablement transfectée avec la chimiokine CCL5 (Rantes) et la molécule d'adhésion ICAM-1 pour optimiser l'infiltration de la tumeur par les lymphocytes T et leur capacité à interagir avec les cellules cibles, et maintenue dans des souris nude (Franciszkiewicz et al., Cancer Res., 2009, 69, 6249-6255). 10 jours plus tard, lorsque les tumeurs sont palpables, 2.10$^7$ cellules mononucléées du sang périphérique (PBMC) de donneurs sains allogéniques humains, préalablement testées *in vitro* pour leur capacité à induire des réponses en cellules T CD8 dirigées contre les peptides antigéniques ppCT, ont été injectées aux souris par voie intraveineuse dans la queue. De l'IL-15 recombinante (3 $\mu$g/souris/jour) a ensuite été administrée en intrapéritonéal pour favoriser la survie des cellules T.

### Peptides

Les peptides utilisés sont :

- la combinaison C6 constituée des peptides ppCT9-17, ppCT16-25 et ppCT50-59 qui comprennent des épitopes restreints à HLA-A et des peptides longs ppCT1-15 et ppCT86-100, et
- la combinaison C6a comprenant en plus des peptides de la combinaison C6, trois peptides restreints à HLA-B7 : ppCT7-15 (SEQ ID NO : 19), ppCT46-54 (SEQ ID NO : 20) et ppCT79-88 (SEQ ID NO : 21).

### Immunisation des souris et suivi de la croissance tumorale

Des souris NSG ayant reçu une greffe de tumeur Heu-nIR et un transfert adoptif de PBMC ont été vaccinées 2 fois à une semaine d'intervalle, avec 100 $\mu$l de la combinaison peptidique C6 ou C6a contenant un mélange équimolaire de chacun des peptides et d'adjuvant (25 ug de poly (I:C), un jour après le transfert de PBMC. Pour la thérapie combinée, les souris vaccinées ont été traitées par voie intraveineuse avec un anticorps anti-hPD-1 (220 mg/injection) ou un anticorps contrôle de même isotype (220 mg de hIgG4 non-relevante), tous les deux jours à partir du deuxième jour après le transfert de PBMC.

Le poids des souris a été suivi tous les jours et les tumeurs ont été mesurées tous les 2 jours pendant seulement 3 ou 4 semaines pour éviter la réaction du greffon contre l'hôte (GVHD). Les échantillons de plasma ont été prélevés le dernier jour de l'expérience, avant le sacrifice des souris et conservés à -80°C jusqu'à leur utilisation. Les souris ont été sacrifiées et les tumeurs pesées puis dissociées pour l'analyse des lymphocytes T infiltrants (TIL) par cytométrie en flux. En parallèle, la rate a été prélevée et la capacité des splénocytes à sécréter de l'interféron gamma (IFN-γ) après stimulation *ex vivo* avec la combinaison de peptides a été testée.

**Tests ELISA**

Les taux d'IFN-γ humain dans le plasma des souris vaccinées et non-vaccinées ont été mesurés à l'aide d'un kit ELISA du commerce (Human IFN-γ EIISA Set ; BD OptEIA™, BD Biosciences), selon les recommandations du fabricant. Tous les échantillons ont été mesurés en duplicat.

**Anticorps et analyses en immunofluorescence**

Les anticorps utilisés sont les anticorps monoclonaux anti-CD45, CD3, CD8, CD4, CD44, PD-1, Perforin, Granzyme B et IFN-γ humains, ainsi que les contrôles isotypique de souris et de lapin (Miltenyi) et les anticorps monoclonaux anti-CD69, CD62L, CD49a, CD45RO et CCR7 humains (Invitrogen). Les expériences de blocage ont été réalisées avec l'anticorps monoclonal anti-PD-1 humain et l'anticorps contrôle non- relevant de même isotype. Les analyses phénotypiques ont été réalisées par immunofluorescence directe, à l'aide d'un cytomètre en flux BD™ LSR II. Les données ont été analysées à l'aide du logiciel FlowJo®.

**Analyses statistiques**

Les analyses statistiques ont été réalisées à l'aide du logiciel Prism 6.0 (logiciel GraphPad). Les résultats des différents groupes ont été comparés à l'aide du test T pour des échantillons indépendants. Une valeur de $P < 0,05$ pour un test bilatéral est considérée comme statistiquement significative.

**RESULTATS**

L'effet anti-tumoral du vaccin peptidique à base de peptides antigéniques de la préprocalcitonine a été évalué dans un modèle de souris humanisées NOD-*scid Il2rγ$^{null}$* (NSG). Pour inverser l'épuisement des lymphocytes T activés et potentialiser d'avantage l'effet thérapeutique bénéfique du vaccin contre le cancer, cette stratégie d'immunothérapie active a été combinée avec un anticorps monoclonal anti-PD1 utilisé en clinique. Les souris NSG ont reçu une greffe de tumeur Heu-nIR puis un transfert adoptif de PBMC de donneurs sains HLA-A2+ et ont ensuite été immunisées avec le vaccin à base de la combinaison C6 ou C6a, seul ou combiné avec un inhibiteur du point de contrôle immunitaire PD-1.

Les résultats montrent que le traitement des souris humanisées avec l'immunothérapie combinée induit une réduction beaucoup plus forte de la croissance tumorale que chacune des monothérapies (Figure 7A). Le contrôle de la croissance tumorale est corrélé avec une réduction du poids des tumeurs chez les souris traitées avec la thérapie combinée, comparé aux souris traitées avec le vaccin ou l'anti-PD1 seuls (Figure 7B).

La production d'IFN-γ humain par les trois groupes de souris a ensuite été évaluée pour analyser si la réduction de la croissance tumorale était associée à une augmentation de la réponse immunitaire. Une augmentation d'un facteur 2 des taux plasmatiques d'IFN-γ humain a été détectée dans le sérum des souris traitées avec l'immunothérapie combinée, comparé aux souris traitées avec chacune des thérapies seules (Figure 7C). En outre, la coloration intracellulaire des splénocytes des souris traitées avec la thérapie combinée indique que les cellules T CD8+ expriment des niveaux plus élevés d'IFN-γ, de granzyme B et de perforine (Figure 7D). Ces résultats indiquent que, comparé aux deux monothérapies, la thérapie combinée est plus efficace pour controler la croissance des tumeurs et que cet effet est corrélé avec des fonctions plus efficaces des cellules T.

L'effet antitumoral de la thérapie combiné est observé avec les deux combinaisons peptidiques testées ; la combinaison C6 qui comprend des peptides restreints à HLA-A2 et de peptides longs (Figures 7A et 7B) et la combinaison C6a qui en plus des peptides de la combinaison C6 comprend également des peptides restreints à HLA-B7 (Figure 8A et 8B).

L'approche combinée induit une augmentation de la sécrétion d'IFN-γ par les cellules T CD8 et de l'infiltration de la tumeur par des cellules T activées, corrélées à une réduction de la croissance tumorale. Par conséquent, la thérapie combinée représente une stratégie intéressante pour traiter les tumeurs qui ont échappé à l'immunité des cellules T CD8 ou aux immunothérapies conventionnelles.

**Revendications**

1. Composition **caractérisée en ce qu'**elle comprend une combinaison de séquences peptidiques choisie parmi la combinaison des séquences SEQ ID Nos : 1, 2, 3, 5 et 6, la combinaison des séquences SEQ ID Nos : 1, 3, 4, 5 et 6 et la combinaison des séquences SEQ ID Nos :1, 3, 5 et 6, chacune desdites séquences étant présente sous forme d'un peptide de 9 à 15 acides aminés ou incluse dans un polypeptide multiépitopique chimérique.

2. Composition selon la revendication 1, **caractérisée en ce qu'**elle comprend un mélange de peptides de 9 à 15 acides aminés.

3. Composition selon la revendication 1 ou la revendication 2, **caractérisée en ce qu'**elle comprend un polypeptide chimérique comprenant ladite combinaison de séquences peptidiques, lesdites séquences étant adjacentes, liées par un élément de liaison constitué par 1 à 5 acides aminés, ou séparées par une séquence comprenant un épitope d'une protéine autre que la préprocalcitonine.

4. Composition comprenant au moins une molécule d'acide nucléique codant pour un mélange de peptides tel que défini à la revendication 2 ou codant pour un polypeptide chimérique tel que défini à la revendication 3.

5. Composition selon l'une quelconque des revendications 1 à 4, comprenant des cellules présentatrices de l'antigène chargées avec lesdites séquences peptidiques.

6. Composition selon l'une quelconque des revendications 1 à 5, comprenant en outre, au moins un inhibiteur de point de contrôle immunitaire, et/ou l'une des séquences peptidiques SEQ ID NO : 19, 20 et/ou 21.

7. Composition selon l'une quelconque des revendications 1 à 6, pour utilisation comme médicament.

8. Composition selon l'une quelconque des revendications 1 à 6, pour utilisation en immunothérapie anti-tumorale.

9. Composition pour utilisation selon la revendication 8, **caractérisée en ce que** ladite composition est destinée à l'immunothérapie de tumeurs exprimant la calcitonine, ou de pathologies associées à un taux sérique élevé de calcitonine, de précalcitonine, ou de prépro-$\alpha$-CGRP.

10. Composition pour utilisation selon la revendication 8 ou la revendication 9, **caractérisée en ce que** ladite composition est destinée à l'immunothérapie d'un carcinome médullaire de la thyroïde ou d'un carcinome pulmonaire.

11. Composition pour utilisation selon l'une quelconque des revendications 8 à 10, **caractérisée en ce que** ladite composition est destinée à l'immunothérapie d'une tumeur dont les cellules n'expriment pas les transporteurs de peptides TAP.

12. Composition pour utilisation selon l'une quelconque des revendications 7 à 11, **caractérisée en ce que** ladite composition est destinée au traitement d'un patient HLA-A*0201.

**Patentansprüche**

1. Zusammensetzung, **dadurch gekennzeichnet, dass** sie eine Kombination von Peptidsequenzen umfasst, die aus der Kombination der Sequenzen SEQ ID NO: 1, 2, 3, 3, 3, 5, 5 und 6, der Kombination der Sequenzen SEQ ID NO: 1, 3, 4, 5 und 6 und der Kombination der Sequenzen SEQ ID NO: 1, 3, 5 und 6 gewählt sind, wobei jede der Sequenzen als Peptid mit 9 bis 15 Aminosäuren vorhanden oder in einem chimären multiepitopischen Polypeptid enthalten ist.

2. Zusammensetzung nach Anspruch 1, **dadurch gekennzeichnet, dass** sie eine Mischung aus Peptiden mit 9 bis 15 Aminosäuren umfasst.

3. Zusammensetzung nach Anspruch 1 oder Anspruch 2, **dadurch gekennzeichnet, dass** sie ein chimäres Polypeptid umfasst, welches die Kombination von Peptidsequenzen umfasst, wobei die Sequenzen benachbart sind, durch ein Bindungselement verbunden sind, das 1 bis 5 Aminosäuren umfasst, oder durch eine Sequenz getrennt sind, die ein Epitop eines anderen Proteins als Präprocalcitonin umfasst.

4. Zusammensetzung, umfassend mindestens ein Nukleinsäuremolekül, das für eine Peptidmischung nach Anspruch 2 oder für ein chimäres Polypeptid nach Anspruch 3 kodiert.

5. Zusammensetzung nach einem der Ansprüche 1 bis 4, umfassend antigenpräsentierende Zellen, die mit den Peptidsequenzen geladen sind.

6. Zusammensetzung nach einem der Ansprüche 1 bis 5, ferner umfassend mindestens einen Immunkontrollpunktinhibitor und/oder eine der Peptidsequenzen SEQ ID NO: 19,20 und/oder 21.

7. Zusammensetzung nach einem der Ansprüche 1 bis 6 zur Verwendung als Medikament.

8. Zusammensetzung nach einem der Ansprüche 1 bis 6 zur Verwendung in der Anti-Tumor-Immuntherapie.

9. Zusammensetzung zur Verwendung nach Anspruch 8, **dadurch gekennzeichnet, dass** die Zusammensetzung für die Immuntherapie von Calcitonin exprimierenden Tumoren oder von Erkrankungen bestimmt ist, die mit erhöhten Serumwerten für Calcitonin, Precalcitonin oder Prepro-$\alpha$-CGRP verbunden sind.

10. Zusammensetzung zur Verwendung nach Anspruch 8 oder Anspruch 9, **dadurch gekennzeichnet, dass** die Zusammensetzung für die Immuntherapie eines medullären Karzinoms der Schilddrüse oder eines Lungenkarzinoms bestimmt ist.

11. Zusammensetzung zur Verwendung nach einem der Ansprüche 8 bis 10, **dadurch gekennzeichnet, dass** die Zusammensetzung zur Immuntherapie eines Tumors dient, dessen Zellen keine TAP-Peptidträger exprimieren.

12. Zusammensetzung zur Verwendung nach einem der Ansprüche 7 bis 11, **dadurch gekennzeichnet, dass** die Zusammensetzung für die Behandlung eines HLA-A*0201-Patienten bestimmt ist.

## Claims

1. Composition **characterised in that** it comprises a combination of peptide sequences chosen from the combination of sequences of SEQ ID NOs: 1, 2, 3, 5 and 6, the combination of sequences of SEQ ID NOs: 1, 3, 4, 5 and 6, and the combination of sequences of SEQ ID NOs: 1, 3, 5 and 6, each of said sequences being present in the form of a peptide of 9 to 15 amino acids or included in a multiepitope chimeric polypeptide.

2. Composition according to claim 1, **characterised in that** it comprises a mixture of peptides of 9 to 15 amino acids.

3. Composition according to claim 1 or claim 2, **characterised in that** it comprises a chimeric polypeptide comprising said combination of peptide sequences, said sequences being adjacent, linked by a linking element consisting of 1 to 5 amino acids, or separated by a sequence comprising an epitope of a protein other than preprocalcitonin.

4. Composition comprising at least one nucleic acid molecule coding for a mixture of peptides as defined in claim 2 or coding for a chimeric polypeptide as defined in claim 3.

5. Composition according to any one of claims 1 to 4, comprising antigen-presenting cells loaded with said polypeptide sequences.

6. Composition according to any one of claims 1 to 5, further comprising at least one immune checkpoint inhibitor, and/or one of the peptide sequences of SEQ ID NO: 19, 20 and/or 21.

7. Composition according to any one of claims 1 to 6, for use as a medicament.

8. Composition according to any one of claims 1 to 6, for use in antitumour immunotherapy.

9. Composition for use according to claim 8, **characterised in that** said composition is intended for immunotherapy of tumours expressing calcitonin, or of pathologies associated with a high serum level of calcitonin, precalcitonin, or prepro-$\alpha$-CGRP.

**10.** Composition for use according to claim 8 or claim 9, **characterised in that** said composition is intended for immunotherapy of a medullary thyroid carcinoma or pulmonary carcinoma.

**11.** Composition for use according to any one of claims 8 to 10, **characterised in that** said composition is intended for immunotherapy of a tumour having cells which do not express TAP peptide transporters.

**12.** Composition for use according to any one of claims 7 to 11, **characterised in that** said composition is intended for the treatment of an HLA-A*0201 patient.

Gène calcitonine/α-CGRP (X15943) chromosome

ARNm α-CGRP

phase ouverte de lecture α-CGRP

ARNm Calcitonine

phase ouverte de lecture Calcitonine

Peptide Signal | 57 aa | Calcitonine | 27 aa

ppCT$_{1-15}$  ppCT$_{16-25}$                                      ppCT$_{71-85}$   ppCT$_{86-100}$

MGFQKFSPFLALSILVLLQAGSLHAAPFRSALESSPADPATLSEDEARLLLAALVQDYVQMKASELEQEQEREGSSLDSPRSKRCGNLSTCMLGTYTQDFNKFHTFPQTAIGVGAPGKKRDMSSDLERDHRPHVSMPQNAN

ppCT$_{9-17}$                                   ppCT$_{50-59}$                                        ppCT$_{91-100}$

*Fig.1*

EP 3 319 625 B1

*Fig.2A*

*Fig. 2B*

*Fig. 2C*

**Combinaison 1** : 3 peptides de 15 aa: ppCT$_{1-15}$ ppCT$_{71-85}$ ppCT$_{86-100}$

ppCT$_{1-15}$                                                                                          ppCT$_{71-85}$        ppCT$_{86-100}$

MGFQKFSPFLALSIL|VLLQAGSLHAAPFRSALESSPADPATLSEDEARLLLAALVQDYVQMKASELEQ|EQEREGSSLDSPRSKR|CGNLSTCMLGTYTQ|DFNKFHTFPQTAIGVGAPGKKRDMSSDLERDHRPHVSMPQNAN

**Combinaison 2** : 4 peptides, 2 de 15 aa et 2 de 10 aa: ppCT$_{1-15}$ ppCT$_{86-100}$ ppCT$_{16-25}$ ppCT$_{50-59}$

ppCT$_{1-15}$ ppCT$_{16-25}$                                                                      ppCT$_{86-100}$

MGFQKFSPFLALSIL|VLLQAGSLHAAPFRSALESSPADPATLSEDEARLLLAALVQDYVQMKASELEQEQEREGSSLDSPRSKR|CGNLSTCMLGTYTQ|DFNKFHTFPQTAIGVGAPGKKRDMSSDLERDHRPHVSMPQNAN
                                    ppCT$_{50-59}$

**Combinaison 3** : 4 peptides, 2 de 15 aa et 2 de 10 aa: ppCT$_{1-15}$ ppCT$_{71-85}$ ppCT$_{16-25}$ ppCT$_{50-59}$

ppCT$_{1-15}$ ppCT$_{16-25}$                                                                      ppCT$_{71-85}$

MGFQKFSPFLALSIL|VLLQAGSLHAAPFRSALESSPADPATLSEDEARLLLAALVQDYVQMKASELEQ|EQEREGSSLDSPRSKR|CGNLSTCMLGTYTQDFNKFHTFPQTAIGVGAPGKKRDMSSDLERDHRPHVSMPQNAN
                                    ppCT$_{50-59}$

**Combinaison 4** : 4 peptides, 1 de 15 aa et 2 de 10 aa et 1 de 9 aa: ppCT$_{86-100}$ ppCT$_{9-17}$ ppCT$_{16-25}$ ppCT$_{50-59}$

          ppCT$_{16-25}$                                                                                    ppCT$_{86-100}$

MGFQKFSPFLALSILVLLQAGSLHAAPFRSALESSPADPATLSEDEARLLLAALVQDYVQMKASELEQEQEREGSSLDSPRSKR|CGNLSTCMLGTYTQ|DFNKFHTFPQTAIGVGAPGKKRDMSSDLERDHRPHVSMPQNAN
     ppCT$_{9-17}$                                      ppCT$_{50-59}$

**Combinaison 5** : 5 peptides, 3 de 15 aa et 2 de 10 aa: ppCT$_{1-15}$ ppCT$_{71-85}$ ppCT$_{86-100}$ ppCT$_{16-25}$ ppCT$_{50-59}$

ppCT$_{1-15}$ ppCT$_{16-25}$                                                                      ppCT$_{71-85}$        ppCT$_{86-100}$

MGFQKFSPFLALSIL|VLLQAGSLHAAPFRSALESSPADPATLSEDEARLLLAALVQDYVQMKASELEQ|EQEREGSSLDSPRSKR|CGNLSTCMLGTYTQ|DFNKFHTFPQTAIGVGAPGKKRDMSSDLERDHRPHVSMPQNAN
                                    ppCT$_{50-59}$

**Combinaison 6** : 5 peptides, 2 de 15 aa et 2 de 10 aa et 1 de 9 aa: ppCT$_{1-15}$ ppCT$_{86-100}$ ppCT$_{9-17}$ ppCT$_{16-25}$ ppCT$_{50-59}$

ppCT$_{1-15}$ ppCT$_{16-25}$                                                                      ppCT$_{86-100}$

MGFQKFSPFLALSIL|VLLQAGSLHAAPFRSALESSPADPATLSEDEARLLLAALVQDYVQMKASELEQEQEREGSSLDSPRSKR|CGNLSTCMLGTYTQ|DFNKFHTFPQTAIGVGAPGKKRDMSSDLERDHRPHVSMPQNAN
     ppCT$_{9-17}$                                      ppCT$_{50-59}$

*Fig. 3*

EP 3 319 625 B1

Fig. 4A

Fig. 4B

*Fig. 4C*

EP 3 319 625 B1

*Fig.5A*

Fig.5B'

Fig. 5B"

**Fig. 5B'''**

**Fig. 6**

*Fig. 7A*

*Fig. 7B*

*Fig. 7C*

Fig. 7D

EP 3 319 625 B1

*Fig. 8A*

*Fig. 8B*

## RÉFÉRENCES CITÉES DANS LA DESCRIPTION

**Documents brevets cités dans la description**

- WO 2009010874 A
- WO 0175179 A
- WO 1999003499 A

**Littérature non-brevet citée dans la description**

- **MORRIS et al.** *Nature,* 1984, vol. 308, 746-8
- **ROSENFELD et al.** *Nature,* 1983, vol. 304, 129-35
- **ZAIDI et al.** *Crit Rev Clin Lab Sci,* 1990, vol. 28, 109-74
- **COOMBES et al.** *Lancet,* 1974, vol. 1, 1080-3
- **MILHAUD et al.** *Lancet,* 1974, vol. 1, 462-3
- **SCHOTT et al.** *Cancer Immunol Immunother,* 2002, vol. 51, 663-8
- **DADAGLIO et al.** *Int. Immunol,* 2003, vol. 15, 1423-1430
- **VIAUD et al.** *J Immunother,* 2011, vol. 34, 65-75
- **TABOLLI et al.** *Tumori,* 1983, vol. 69, 227-230
- **SIM et al.** *Ann Clin Lab Sci,* 1996, vol. 26, 487-95
- **CONTE et al.** *Acta Endocrinol,* 1984, vol. 106, 109-11
- **TAKUBO et al.** *Haematologia,* 2001, vol. 31, 177-9
- **KIEFER et al.** *Leuk Lymphoma,* 1994, vol. 13, 501-507
- **GHILLIANI et al.** *Cancer Res,* 1989, vol. 49, 6845-6851
- **FRANCISZKIEWICZ et al.** *Cancer Res.,* 2009, vol. 69, 6249-6255